(19) Europäisches Patentamt
European Patent Office
Office européen des brevets

(11) **EP 2 599 464 B1**

(12) **EUROPÄISCHE PATENTSCHRIFT**

(45) Veröffentlichungstag und Bekanntmachung des Hinweises auf die Patenterteilung:
**18.01.2017   Patentblatt 2017/03**

(51) Int Cl.:
***A61F 2/80*** *(2006.01)*

(21) Anmeldenummer: **12007785.4**

(22) Anmeldetag: **16.11.2012**

(54) **Prothese**

Prosthesis

Prothèse

(84) Benannte Vertragsstaaten:
**AL AT BE BG CH CY CZ DE DK EE ES FI FR GB GR HR HU IE IS IT LI LT LU LV MC MK MT NL NO PL PT RO RS SE SI SK SM TR**

(30) Priorität: **29.11.2011   DE 102011119591**

(43) Veröffentlichungstag der Anmeldung:
**05.06.2013   Patentblatt 2013/23**

(73) Patentinhaber: **Otto Bock HealthCare GmbH 37115 Duderstadt (DE)**

(72) Erfinder:
• **Mosler, Lüder**
  **37115 Duderstadt (DE)**
• **Weber, Scott**
  **56303 St. Cloud Minnesota (US)**

(74) Vertreter: **Friedrich, Andreas et al Gramm, Lins & Partner Patent- und Rechtsanwälte PartGmbB Theodor-Heuss-Straße 1 38122 Braunschweig (DE)**

(56) Entgegenhaltungen:
**US-A1- 2003 181 990     US-A1- 2007 191 965**

## Beschreibung

Die Erfindung betrifft eine Prothese mit einem Prothesenschaft, der eine Innenfläche aufweist und ausgebildet ist, an einem Amputationsstumpf angeordnet zu werden, so dass die Innenfläche dem Amputationsstumpf zugewandt ist und zwischen der Innenfläche und dem Amputationsstumpf ein Volumen eingeschlossen ist, und einer Pumpe zum Erzeugen eines Unterdruckes in dem Volumen, wenn der Prothesenschaft an dem Amputationsstumpf angeordnet ist.

Eine derartige Prothese ist beispielsweise aus der US 2007/0191965 A1 bekannt.

Derartige Prothesen sind heute aus dem Stand der Technik zur Genüge bekannt. Über den Amputationsstumpf wird oftmals zunächst ein Liner gezogen. Dabei kann es sich beispielsweise um ein Silikonmaterial oder ein Polyurethanmaterial handeln, das an die individuelle Form des jeweiligen Amputationsstumpfes angepasst wurde. Über diesen Liner wird dann der Prothesenschaft der Prothese gestülpt, so dass die Innenseite des Prothesenschaftes dem Liner und damit dem Amputationsstumpf zugewandt ist. Wird kein separater Liner verwendet, ist die Innenseite des Prothesenschaftes direkt dem Amputationsstumpf zugewandt. Alternativ kann beispielsweise auch ein Sleeve über den Amputationsstumpf gezogen werden, der den oberen also proximalen Rand des Prothesenschaftes gegen den Amputationsstumpf luftdicht abdichtet. Um den Prothesenschaft und damit die Prothese an der gewünschten Stelle zu fixieren und dies auch beispielsweise bei starker Bewegung des Patienten zu gewährleisten, ist es aus dem Stand der Technik bekannt, zwischen dem Liner und dem Prothesenschaft einen Unterdruck zu erzeugen, durch den der Prothesenschaft in seiner Position gehalten wird. Dafür sind unterschiedlichste Anordnungen bekannt.

In der Regel verfügt der Prothesenschaft über eine distale Durchgangsbohrung, an der eine Vakuumpumpe angeschlossen ist. Eine derartige Anordnung ist beispielsweise aus der WO 2006/135851 A2 bekannt. Dabei ist die Vakuumpumpe zusammen mit einer dafür vorgesehenen Stromversorgung Teil des Prothesenaufbaus. Wird eine derartige Prothese über einen längeren Zeitraum, beispielsweise einen Tag, getragen, ist es möglich, dass aufgrund der Bewegung des Amputationsstumpfes und des Prothesenschaftes sowie durch kleine Undichtigkeiten Luft in das Volumen zwischen dem Liner und der Innenfläche des Prothesenschaftes eindringen kann. Dadurch wird der Unterdruck gemindert, wodurch die Befestigung der Prothese am Amputationsstumpf geschwächt wird.

Für diesen Fall hat der Träger einer in der WO 2006/135851 A2 beschriebenen Prothese die Vakuumpumpe als Teil der Prothese immer bei sich, so dass er bei nachlassendem Unterdruck die Pumpe erneut aktivieren und den Unterdruck auf die gewünschte Stärke einstellen kann.

Nachteilig ist, dass eine derartige Pumpe, die häufig als Membranpumpe ausgebildet ist, platz- und gewichtsintensiv ist, so dass sie gegebenenfalls nicht in jeder Prothese unterbringbar ist. Zudem muss für eine ausreichende Stromversorgung beispielsweise in Form von Batterien gesorgt werden. Hinzu kommt, dass Membranpumpen um Ventile ergänzt werden müssen, die bei einer Verschmutzung die zuverlässige Pumpenfunktion beeinträchtigen können. Um eine Membranpumpe sowohl als Vakuumpumpe als auch als Ausstoßpumpe verwenden zu können, sind zusätzlich aufwändige Ventilschaltungen nötig.

Um diesen Nachteil aus dem Weg zu gehen, ist es beispielsweise aus der US 5,702,489 und der US 6,926,742 bekannt, eine externe Vakuumpumpe vorzusehen. Der Nachteil bei diesem System besteht jedoch darin, dass der Träger der Prothese die Pumpe immer als separates Bauteil mit sich herumtragen muss, um bei einem eventuellen Druckverlust zwischen dem Liner und dem Prothesenschaft die Pumpe anschließen zu können. Zudem muss er die Vakuumpumpe dann an eine entsprechende Vorrichtung, die beispielsweise am Prothesenschaft vorgesehen sein kann, anschließen, was insbesondere für ältere oder in ihrer Mobilität eingeschränkte Träger von Prothesensystemen schwierig bis unmöglich ist.

Unabhängig davon, ob bei einer Prothese eine externe oder eine interne Vakuumpumpe vorgesehen ist, weisen diese Systeme alle ein Ventilsystem auf, mit dem bei einmal eingestelltem Unterdruck verhindert wird, dass Luft in das Volumen zwischen dem Liner und dem Prothesenschaft eindringen kann. Derartige Ventile umfassen bewegliche Teile und sind daher verschmutzungs- und fehleranfällig und entsprechend wartungsintensiv. Zudem ist die Herstellung relativ aufwändig und kostenintensiv. Hinzu kommt, dass Ventile auch beispielsweise von dem Träger der Prothese aus Versehen oder falsch bedient werden können, so dass Luft in das Volumen zwischen dem Liner und dem Prothesenschaft eindringen kann. In diesem Fall ist eine sichere Anordnung der Prothese am Amputationsstumpf nicht mehr zu gewährleisten.

Aus der US 2003/0181990 A1 ist ein Prothesenschaft bekannt, bei dem sich an der Innenseite ein Kissensystem befindet, das mit einem Fluid befüllbar ist. Über eine vorgesehene Pumpe kann das Volumen der Kissen und der Druck im Inneren der Kissen verändert werden, um so Volumenschwankungen des Amputationsstumpfes auszugleichen.

Der Erfindung liegt daher die Aufgabe zugrunde, eine gattungsgemäße Prothese derart weiterzuentwickeln, dass die Anzahl der beweglichen und damit fehleranfälligen Teile stark reduziert, eine Fehlbedienung nahezu ausgeschlossen und dennoch eine platz-, energie- und kostengünstige Lösung für das Bereitstellen des nötigen Unterdrucks gefunden wird.

Die Erfindung löst die gestellte Aufgabe durch eine gattungsgemäße Prothese, bei der die Pumpe zum Erzeugen des Unterdrucks in dem Volumen eine peristaltische Pumpe ist. Dabei ist für die Erfindung unerheblich, ob der Prothesenschaft der erfindungsgemäßen Prothese direkt am Amputationsstumpf des Patienten anliegt oder ob eine Zwischenlage, beispielsweise ein Liner, oder eine andere Art des Abschlusses des Volumens vorgesehen ist. Wichtig ist lediglich, dass ein luftdicht abgeschlossenes Volumen definiert ist, in dem ein Unterdruck herstellbar ist, durch den die Prothese am Amputationsstumpf gehalten wird. Dabei ist es auch unerheblich, wie groß dieses Volumen ist, wie weit also in proximaler Richtung der luftdichte Abschluss vom Ansaugpunkt der Pumpe entfernt ist.

Das Pumpenprinzip einer peristaltischen Pumpe beruht darauf, dass ein Medium, vorliegend also die Luft aus dem Volumen zwischen dem Amputationsstumpf oder einem darüber gezogenen Liner und dem Prothesenschaft, durch die mechanische Verformung eines Schlauches durch diesen hindurchgedrückt wird.

In einer besonders bevorzugten Ausgestaltung umfasst die peristaltische Pumpe ein Gehäuse mit einer Umfangswandung, einem Boden und einem Deckel. Innerhalb dieses Gehäuses an der Innenwand der Umfangswandung wird ein Schlauch entlang geführt. Die peristaltische Pumpe verfügt über wenigstens ein, in einer bevorzugten Ausgestaltung über genau ein, Pumpelement. Dieses ist beispielsweise an einer zentral in axialer Richtung, also vom Boden zum Deckel des Gehäuses, in dem Gehäuse laufenden Welle gelagert. Das wenigstens eine, bevorzugt genau eine, Pumpelement, läuft dabei um die Welle um und quetscht an seiner radial äußeren Seite den zwischen ihm und der Innenwand der Umfangswandung des Gehäuses laufenden Schlauch ab. Durch das Fortbewegen des Pumpelementes in Umfangsrichtung wird das in dem Schlauch eingeschlossene Fördermedium durch den Schlauch nach vorne gedrückt. Natürlich sind auch zwei oder mehr Pumpelemente möglich.

Peristaltische Pumpen sind aus dem Stand der Technik seit langem bekannt. Zu ihren Vorteilen zählen eine schonende Förderung von empfindlichem Fördergut, wie beispielsweise Blutzellen, die durch beispielsweise schnell drehende Propellerblätter anderer Pumpentechnologien zerstört würden. Daher werden peristaltische Pumpen, die auch Schlauchpumpen oder Schlauchquetschpumpen genannt werden, insbesondere bei Infusionspumpen und als Blutpumpe in Dialysegeräten und Herzlungenmaschinen eingesetzt. Sie werden immer dann verwendet, wenn ein empfindliches Fördergut über eine lange Zeit gleichmäßig gepumpt werden muss. Daher wurde sie bisher für den vorliegenden Anwendungszweck als nicht geeignet eingestuft.

Dies hat mehrere Gründe. Zum einen wird bei der hier beschriebenen Anwendung einer peristaltischen Pumpe kein Dauerbetrieb benötigt. Vielmehr wird beim Anlegen der Prothese einmal der Unterdruck in dem Volumen zwischen dem Prothesenschaft und dem Liner hergestellt. Sobald der Unterdruck eingestellt ist, wird die Pumpe abgeschaltet und im Optimalfall auch nicht wieder verwendet. Zudem funktionieren peristaltische Pumpen am besten, wenn der Druck im Schlauch gleich oder leicht größer ist als der Druck innerhalb des Gehäuses, aber außerhalb des Schlauches, da sich der Schlauch nach dem Abquetschen des Schlauches durch das wenigstens eine Pumpelement wieder in seine ursprüngliche Form zurückstellen muss, um für die Aufnahme weiteren Fördergutes zur Verfügung zu stehen. Daher ging man davon aus, dass die Herstellung eines Unterdruckes, bei der der Druck innerhalb des Schlauches deutlich geringer ist als der Druck außerhalb des Schlauches aber innerhalb des Gehäuses der Pumpe mit einer derartigen Pumpe nicht zu bewerkstelligen ist. Überraschenderweise stellte sich dies als nicht zutreffend heraus.

Für die Herstellung eines Unterdruckes ist es sinnvoll, dass die Pumpe einen möglichst großen Hub erzeugt, also möglichst wenige Pumpelemente verwendet. Daher umfasst die peristaltische Pumpe in einer besonders bevorzugten Ausführungsform genau ein Pumpelement. Bevorzugt weist die Umfangswandung des Gehäuses der peristaltischen Pumpe eine Öffnung auf, durch die der Schlauch in das Gehäuse hinein und aus dem Gehäuse herausgeführt ist. Dabei ist der Schlauch besonders vorteilhafterweise so geführt, dass es an keiner Stelle zu einer Überlagerung oder Überschneidung des Schlauches mit sich selbst kommt. So ist es beispielsweise sinnvoll, den Schlauch durch die Öffnung in das Gehäuse der peristaltischen Pumpe einzuführen, ihn einmal an der Innenwand der Umfangswandung entlang zu führen und durch die gleiche Öffnung, durch die er in das Gehäuse hineingeführt wurde, auch wieder heraus zu führen. Dabei verläuft der Schlauch direkt nach dem Eintreten in das Gehäuse bzw. direkt vor dem Austreten aus dem Gehäuse in einer sehr scharfen Biegung, um eine Überlagerung oder Überschneidung des Schlauches mit sich selbst zu vermeiden. Ein derartiger "Knick" im Verlauf des Schlauchs ist einer Überlagerung oder Überschneidung vorzuziehen, da eine derartige Überlagerung oder Überschneidung nicht nur zu einer größeren Bauform führte, sondern auch zu einer deutlich erhöhten radialen Kraft auf das wenigstens eine Pumpelement in diesem Bereich. Wird der Schlauch durch eine ovale Öffnung in das Pumpengehäuse hinein und aus diesem heraus geführt, kann die Biegung weniger scharf ausgebildet werden.

Das Pumpelement muss den Schlauch an der schmalsten Stelle zwischen dem einen Pumpelement und der Innenwand der Umfangswandung zusammendrücken. Gibt es jedoch eine oder mehrere Stellen, an denen der Schlauch doppelt geführt ist, an der also eine Überschneidung oder Überlagerung des Schlauches mit sich selbst auftritt, muss das Pumpelement den Schlauch an dieser Stelle zweimal abquetschen. Dadurch entsteht eine deutlich erhöhte Kraft, die auf die zentral laufende Welle übertragen wird. Um diese Kraft möglichst gering zu halten, ist die beschriebene Ausführungsform vorteilhaft. Dennoch muss auch bei dieser Ausführungsform, um eine einwandfreie Funktion der Pumpe gewährleisten zu können, das Pumpelement sowohl den in das Gehäuse eintretende als auch das aus dem Gehäuse austretende Schlauchende abdrücken, um ein Nachströmen von Luft in das bereits gepumpte Vakuum bzw. den bereits gepumpten Unterdruck zu verhindern. Daher ist die Ein- bzw. Ausführung des Schlauches durch eine Öffnung direkt nebeneinander besonders vorteilhaft.

Als ebenfalls vorteilhaft hat sich erwiesen, wenn das Pumpelement eine Rolle ist, die so in dem Gehäuse angeordnet ist, dass sie um die Welle umlaufen kann und den Schlauch abklemmt. Dabei ist vorteilhafterweise der Schlauch an der Innenseite der Umfangswandung entlang geführt und bildet zusammen mit der Rolle und der Welle ein Reibradplanetengetriebe. In diesem Fall ist es nicht erforderlich, ein separates Getriebe zwischen den Pumpenkopf, also der eigentlichen Pumpe, und einen Motor zu schalten, der verwendet wird, um die Pumpe anzutreiben, um das erforderliche Drehmoment aufzubringen. Dieses Getriebe wird durch das beschriebene Reibradplanetengetriebe aus Welle, Rolle und Schlauch ausgeführt. Die Rolle, also das wenigstens eine Pumpelement, wird mit Vorspannung zwischen die Welle, die vom Boden zum Deckel des Gehäuses verläuft, und den Schlauch eingesetzt. Durch diese Vorspannung entsteht ein Reibkontakt zwischen der Rolle und der Welle, durch welchen die Übertragung des Drehmomentes möglich wird. Über das Durchmesserverhältnis von Welle und Rolle kann das Übersetzungsverhältnis geändert und an die jeweils gewünschte Ausführungsform angepasst werden. Wird nun die Welle über die Drehbewegung des Motors in Bewegung gesetzt, wird durch den Reibkontakt das Drehmoment auf die Rolle übertragen, die sich somit entlang des Schlauches um die Welle herum bewegt.

Dazu ist es insbesondere vorteilhaft, wenn das wenigstens eine Pumpelement, also die Rolle, und die Welle mit einem die Reibung erhöhenden Material, beispielsweise einem Kunststoff, beschichtet ist. Alternativ dazu kann beispielsweise auch die Motorwelle oder ein anderes Bauteil aus Edelstahl hergestellt sein, wobei die Oberfläche beispielsweise durch ein Lasererosionsverfahren zumindest an den Stellen aufgeraut wird, an denen die Welle in Kontakt mit der Rolle kommt.

Vorteilhafterweise ist die Rolle in Form einer Hohlrolle ausgebildet. Die Verwendung einer Hohlrolle gewährleistet die Verwendbarkeit der peristaltischen Pumpe und damit der Prothese in einem größeren Temperaturbereich. So ist es beispielsweise möglich, die Wärmeausdehnungen der Rolle in einem Temperaturbereich von beispielsweise -20°C bis +60°C kompensieren zu können. Insbesondere bei der Verwendung eines Reibradplanetengetriebes, wie es oben beschrieben wurde, sind gewisse Toleranzanforderungen an die einzelnen Durchmesser der verwendeten Bauteile erforderlich. Würde die wenigstens eine Rolle in Form einer massiven Vollrolle ausgeführt, würde dies beispielsweise bei niedrigen Temperaturen zu einem Schlupf im Getriebe führen, so dass das notwendige Drehmoment nicht mehr übertragen würde und die Funktionsfähigkeit der peristaltischen Pumpe eingeschränkt wäre. Bei hohen Temperaturen würde die Ausdehnung der Rolle zu erhöhten Biegemomenten auf die Welle und damit auf das Motorlager führen.

Um den Energiebedarf der peristaltischen Pumpe weiter zu senken, ist vorzugsweise an einer Innenseite des Deckels und/oder an einer Innenseite des Bodens des Gehäuses eine reibungsmindernde Schicht aufgebracht, die beispielsweise eine PTFE-Gewebefolie sein kann. Allgemein ist es von Vorteil, wenn die beweglichen Bauteile, also insbesondere die Welle und die wenigstens eine Rolle, möglichst reibungsarm gelagert werden. So ist es beispielsweise sinnvoll, die Welle an beiden Enden in einem Kugellager zu lagern, um die Reibungsverluste zu minimieren.

Der Schlauch besteht vorteilhafterweise aus Silikon oder einem thermoplastischen Elastomer oder aus Polyurethan. Dabei ist das thermoplastische Elastomer vorzuziehen, da es eine deutlich geringere Permeabilität für Luft hat als Silikon. Dadurch dringt weniger Luft durch den Schlauch hindurch, was insbesondere dann von Vorteil ist, wenn die peristaltische Pumpe abgeschaltet ist, da der erforderliche Unterdruck in dem Volumen zwischen dem Liner und dem Prothesenschaft bereits hergestellt wurde. Alternativ sind auch andere Materialien denkbar. So kann der Schlauch auch beispielsweise aus einem Naturkautschuk oder TPE bestehen.

Für den Fall, dass nach dem Anlegen der Prothese durch die peristaltische Pumpe der nötige Unterdruck bereits hergestellt wurde, kann die Pumpe einfach abgeschaltet werden. Auch in diesem Zustand drückt das wenigstens eine Pumpelement den Schlauch sicher ab, so dass ein Eintreten von Luft in das mit dem Unterdruck beaufschlagte Volumen sicher verhindert wird, ohne dass separate und mit beweglichen Teilen versehene Ventile oder gar Ventilsysteme vorgesehen sein müssen. Dadurch wird nicht nur der Herstellungsaufwand und damit die Herstellungskosten reduziert, sondern auch der nötige Bauraum verkleinert, so dass die Pumpanordnung mit der peristaltischen Pumpe auch bei kleineren Prothesen Anwendung finden kann.

Um diesen Effekt noch zu verstärken, kann als Motor zum Antreiben der Pumpe ein Scheibenläufermotor verwendet werden. Diese Motoren sind im Wesentlichen Elektromotoren, deren Rotoren die Form einer Scheibe haben. Sie sind insbesondere auch ohne separaten Eisenkern ausführbar, wodurch sie sehr leicht gebaut werden können und daher ein geringes Trägheitsmoment haben. Sie können daher besonders rasch beschleunigen oder abbremsen. Zudem wirken magnetische Kräfte nur dann auf den Rotor, wenn die Scheibe von Strom durchflossen wird. Der für den vorliegenden Anwendungszweck größte Vorteil eines derartigen Scheibenläufermotors ist neben dem geringen Gewicht der, dass sie auch bei niedrigen Drehzahlen sehr gleichmäßig und ruhig laufen. Es kann folglich auch ein Getriebe zur Untersetzung verzichtet werden, wodurch die Anzahl der beweglichen Teile weiter reduziert und der Kosten- und Montageaufwand ebenfalls weiter verringert werden. Zudem wird der Bauraumbedarf weiter reduziert. Gängige Drehzahlen liegen beispielsweise bei 300 Umdrehungen pro Minute, bevorzugt darunter, etwa bei 150 bis 250 Umdrehungen pro Minute.

In einer bevorzugten Ausführungsform ist an der Innenwand der Umfangswandung des Gehäuses der Pumpe eine Innenkontur vorgesehen, welche bewirkt, dass der darin gelagerte Schlauch in axialer Richtung nicht verrutschen kann, sondern in dieser Position gehalten wird. Bei der Auswahl der Schlauchparameter ist insbesondere darauf zu achten, dass der Schlauch sich auch bei dem relativ großen Druckunterschied zwischen seinem Innenraum und dem Gehäuse wieder aufrichtet, nachdem er durch das eine Pumpelement, also die Rolle, abgequetscht wurde. Dabei sind als Shorehärte für den Schlauch beispielsweise Werte von 60 bis 65 günstig, wenn der Schlauch beispielsweise 5 mm Außendurchmesser bei einer Wandstärke von 1 mm aufweist. Natürlich sind auch Kombinationen größerer Härte mit dünnerer Wandstärke denkbar. Mit einer derartigen peristaltischen Pumpe sind Unterdrücke in dem Volumen zwischen dem Liner und dem Prothesenschaft von bis zu 900 mbar zu erreichen. Wird der Schlauch beispielsweise aus einem thermoplastischen Elastomer hergestellt, besitzt er eine sehr hohe Verschleißfestigkeit, was für die Verwendung in einer peristaltischen Pumpe von Vorteil ist. Beim Pumpen unterliegt der Schlauch einer ständigen Walkbeanspruchung in Form einer mechanischen Wechselbelastung. Zudem verfügt das Material über ein hohes Rückstellvermögen, so dass auch bei hohen Unterdrücken ein Kollabieren des Schlauches vermieden werden kann.

Als vorteilhaft hat sich herausgestellt, wenn der Schlauch einen Querschnitt aufweist, der dafür sorgt, dass beim Abdrücken des Schlauches durch das Zusammenquetschen zwischen dem einen Pumpelement und der Innenwand des Gehäuses möglichst keine oder nur eine sehr geringe Wulstbildung zu beobachten ist. Dies kann beispielsweise dadurch erreicht werden, dass der Schlauch an zwei gegenüberliegenden Seiten im Querschnitt eine geringere Wandstärke aufweist. Alternativ oder zusätzlich dazu sind auch Querschnitte denkbar, die von einem kreisförmigen Querschnitt abweichen.

Um ein leichtes Aufrichten des Schlauches nach dem Abdrücken durch das Pumpelement zu erreichen, kann im Inneren des Gehäuses ein Vakuum vorgesehen sein, wodurch die Druckdifferenz zwischen dem Innenraum des Gehäuses und dem Inneren des Schlauches reduziert wird.

Zusätzlich oder alternativ dazu kann der Schlauch insbesondere innen, aber auch außen, beispielsweise mittels CVD (chemische Gasphasenabscheidung) beschichtet sein, um eine mögliche Haftung der Innenwände des Schlauches aneinander zumindest zu verringern oder ganz zu vermeiden.

Mit Hilfe einer Zeichnung wird nachfolgend ein Ausführungsbeispiel der vorliegenden Erfindung näher erläutert. Es zeigt:

Figur 1 -    die schematische Ansicht einer peristaltischen Pumpe für eine Prothese gemäß einem ersten Ausführungsbeispiel der vorliegenden Erfindung,
Figur 2 -    die Pumpe aus Figur 1 in einer schematischen Draufsicht,
Figur 3 -    eine weitere Ansicht einer peristaltischen Pumpe,
Figur 4 -    eine weitere Ansicht einer peristaltischen Pumpe,
Figur 5 -    eine schematische 3D-Ansicht einer peristaltischen Pumpe für eine Prothese gemäß einem weiteren Ausführungsbeispiel der vorliegenden Erfindung,
Figur 6 -    die Pumpe aus Figur 5 in einer schematischen Seitenansicht,
Figur 7 -    eine schematische Ansicht einer geöffneten peristaltischen Pumpe,
Figur 8 -    eine weitere schematische Ansicht einer geöffneten peristaltischen Pumpe,
Figur 9 -    eine schematische Schnittansicht durch eine peristaltische Pumpe,
Figur 10 -   eine weitere Schnittdarstellung durch eine peristaltische Pumpe für eine Prothese gemäß einem Ausführungsbeispiel der vorliegenden Erfindung,
Figur 11 -   die schematische Darstellung zweier bevorzugter Schlauch-Querschnitte,
Figur 12 -   die schematische Seitenansicht einer peristaltischen Pumpe gemäß einem Ausführungsbeispiel der vorliegenden Erfindung,
Figur 13 -   zeigt eine schematische Darstellung eines Schlauchquerschnittes,
Figur 14 -   zeigt eine schematische Darstellung eines aufeinander gelegten und eines zusammengepressten Schlauches,
Figur 15 -   zeigt eine schematische Seitenansicht eines Pumpengehäuses,
Figur 16 -   zeigt eine schematische Draufsicht auf ein Pumpengehäuse mit entferntem Deckel,
Figur 17 -   zeigt eine schematische 3D-Ansicht eines Prothesenschaftes mit einer daran angebrachten Pumpe,
Figur 18 -   zeigt die Darstellung aus Figur 17 aus einem anderen Blickwinkel,
Figur 19 -   zeigt eine schematische Darstellung einer Prothese gemäß einem Ausführungsbeispiel der vorliegenden Erfindung an einem Amputationsstumpf und
Figur 20 -   zeigt die Darstellung aus Figur 19 aus einem anderen Blickwinkel.

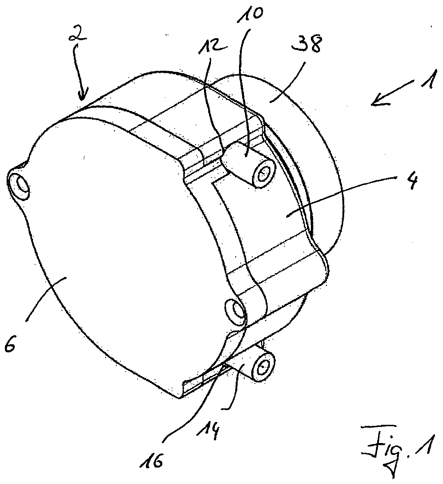

Figur 1 zeigt eine peristaltische Pumpe 1, die ein Gehäuse 2 aufweist, das über eine Umfangswandung 4, einen Deckel 6 und einen nicht gezeigten Boden 8 verfügt. In Figur 1 ist ein erstes Schlauchende 10 gezeigt, das durch eine erste Öffnung 12, die sich in der Umfangswandung 4 des Gehäuses 2 befindet, in das Gehäuse 2 eingeführt ist. Ein zweites Schlauchende 14 ist durch eine zweite Öffnung 16 aus dem Gehäuse 2 herausgeführt. Am Boden 8 ist ein Motor 38 angeordnet.

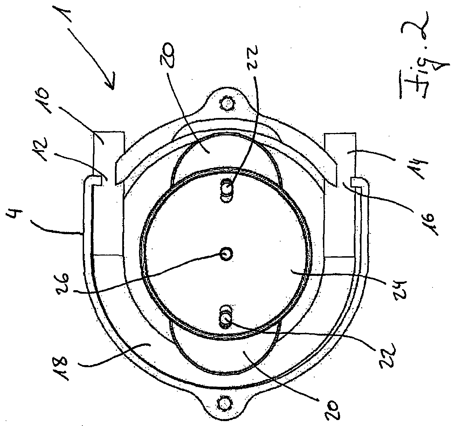

Figur 2 zeigt eine schematische Draufsicht auf eine geöffnete peristaltische Pumpe 1 gemäß Figur 1. Man erkennt die Umfangswandung 4 sowie das erste Schlauchende 10 und das zweite Schlauchende 14, die durch eine erste Öffnung 12 bzw. eine zweite Öffnung 16 durch die Umfangswandung 4 geführt werden. In dem Gehäuse 2 selbst verläuft ein Schlauch 18 entlang der Umfangswandung 4. In der in Figur 2 gezeigten Ausführungsform verfügt die peristaltische Pumpe 1 über zwei Pumpelemente 20. Die beiden Pumpelemente 20 sind in Form von Rollen ausgebildet. Diese sind um eine jeweilige Rollenachse 22 drehbar gelagert. Die Rollenachsen 22 erstrecken sich im in Figur 2 gezeigten Ausführungsbeispiel senkrecht zur Zeichenebene. Sie sind in einer Abstandsscheibe 24 gelagert, die über eine Welle 26 verfügt, über die sie mittels eines nicht gezeigten Motors in Rotation versetzbar ist. Man erkennt am in Figur 2 linken Bildrand, dass das links gezeigte Pumpelement 20 den Schlauch 18 zwischen sich und der Umfangswandung 4 abquetscht. Wird nun die Welle 26 in Rotation versetzt, beispielsweise mit dem Uhrzeigersinn, bewegen sich die beiden Pumpelemente 20 entgegen dem Uhrzeigersinn. Dabei schiebt das in Figur 2 linke Pumpelement 20 ein sich im Schlauch 18 befindliches Medium in Richtung auf das zweite Schlauchende 14. Bevor das in Figur 2 linke Pumpelement 20 den Schlauch 18 wieder freigibt, drückt das in Figur 2 rechte Pumpelement 20 den Schlauch 18 in der Nähe der ersten Öffnung 12 wieder ab, so dass ein Rückfließen des Mediums durch das erste Schlauchende 18 unmöglich ist. Durch die Abstandsscheibe 24 wird gewährleistet, dass beide Pumpelemente 20 immer gegenüber voneinander angeordnet sind. Durch die vorgesehenen Langlöcher können Toleranzen und beispielsweise die Wärmeausdehnung ausgeglichen werden.

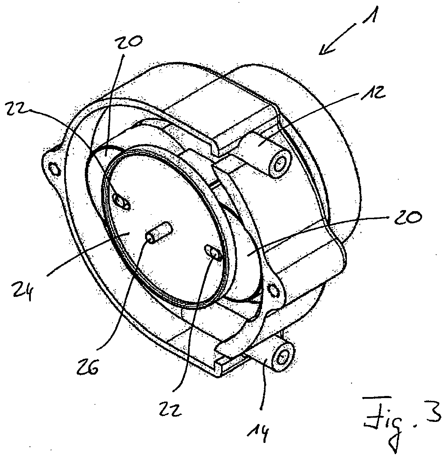

Figur 3 zeigt die Ansicht aus Figur 2 aus einem anderen Blickwinkel.

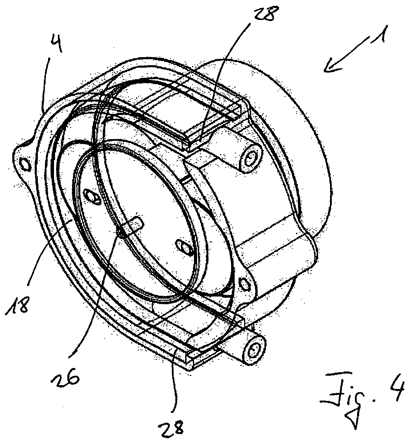

Figur 4 zeigt die in den Figuren 2 und 3 gezeigte peristaltische Pumpe 1, wobei nun an der Innenwandung der Umfangswandung 4 eine Führung 28 angeordnet ist, die beispielsweise aus einem Silikon bestehen kann. Diese ist in Form einer umlaufenden Rille ausgebildet, in der der Schlauch 18 angeordnet ist. Dadurch wird ein Verrutschen bzw. ein Verschieben des Schlauchs 18 in bezüglich der Welle 26 axialer Richtung verhindert. Zudem werden Fertigungstoleranzen sowie die Wärmeausdehnung der einzelnen Bauteile ausgeglichen.

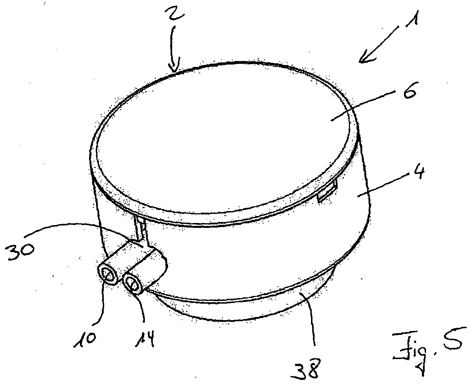

Figur 5 zeigt eine schematische 3D-Ansicht einer peristaltischen Pumpe 1 für eine Prothese gemäß einem weiteren Ausführungsbeispiel der vorliegenden Erfindung. Auch diese peristaltische Pumpe weist das Gehäuse 2 auf, das über die Umfangswandung 4, den Deckel 6 und den Boden 8 verfügt, an dem der Motor 38 angeordnet ist. Man erkennt jedoch, dass in der Umfangswandung 4 nur eine Öffnung 30 vorgesehen ist, durch die sowohl das erste Schlauchende 10 als auch das zweite Schlauchende 14 geführt ist.

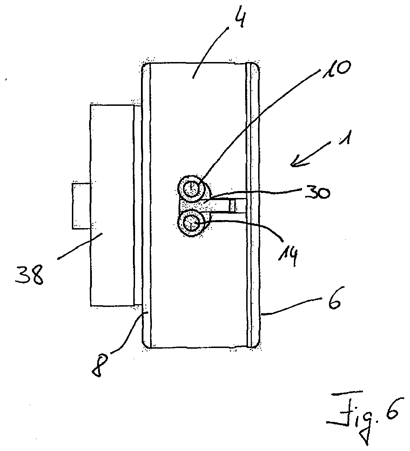

Figur 6 zeigt die Darstellung aus Figur 5 in einer Seitenansicht. Man erkennt das erste Schlauchende 10 und das zweite Schlauchende 14, die unmittelbar nebeneinander durch die Öffnung 30 geführt sind. Auch wenn in Figur 6 ein kleiner Zwischenraum zwischen dem ersten Schlauchende 10 und dem zweiten Schlauchende 14 dargestellt ist, ist es auch möglich und insbesondere vorteilhaft, beide Schlauchenden 10, 14 direkt nebeneinander, also einander berührend, aus der Öffnung 30 heraus zu führen.

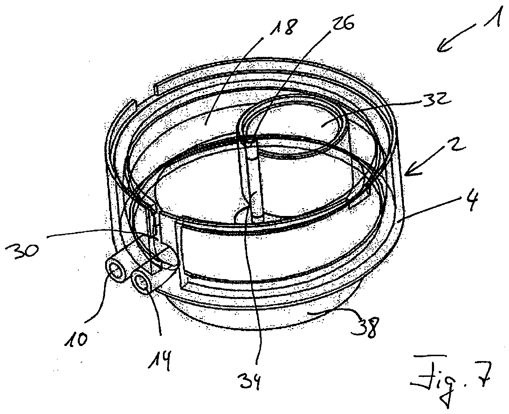

Figur 7 zeigt die Darstellung einer geöffneten peristaltischen Pumpe gemäß den Figuren 5 und 6, bei der der Deckel 6 entfernt wurde. Das erste Schlauchende 10 gelangt durch die Öffnung 30 in das Gehäuse 2 der peristaltischen Pumpe 1. Im in Figur 7 gezeigten Ausführungsbeispiel macht der Schlauch 18 eine Linkskurve und wird einmal an der Innenwand der Umfangswandung 4 entlang geführt. Sobald der Schlauch 18 wieder die Öffnung 30 erreicht, wird das zweite Schlauchende 14 durch die Öffnung 30 wieder heraus geführt.

Im Inneren des Gehäuses 2 befindet sich eine Welle 26 sowie eine Rolle 32, die das einzige Pumpelement der in Figur 7 gezeigten peristaltischen Pumpe 1 darstellt. Die Rolle 32 ist als Hohlrolle ausgeführt, um der Ausdehnung bzw. dem Zusammenziehen bei Temperaturschwankungen Rechnung zu tragen. Die Welle 26 verfügt über einen aufgerauten Bereich 34, der genau der Bereich ist, in dem die Welle 26 mit der Rolle 32 in Kontakt kommt. Alternativ oder zusätzlich kann die Welle 26 auch geriffelt oder gezahnt oder mit einer anderen Struktur versehen sein, durch die die Reibung zwischen Welle 26 und Rolle 32 erhöht wird. Die Mantelfläche der Rolle 32 kann ebenfalls aufgeraut oder mit einem die Reibung erhöhenden Material beschichtet sein. Man erkennt, dass die Rolle 32, anders als in den Figuren 2, 3 und 4 dargestellt, nicht über eine Rollenachse verfügt. Stattdessen wird das nötige Drehmoment von der Welle 26, die über einen nicht gezeigten Motor antreibbar ist, allein durch den Reibkontakt zwischen der Welle 26 und der Rolle 32 auf die Rolle 32 übertragen. Die Rolle 32 klemmt den Schlauch 18 zwischen sich und der Innenwand der Umfangswandung 4 ein und drückt ihn so ab. Die Welle 26, die Rolle 32 und der Schlauch 18 bilden so einen Reibradplanetengetriebe. Dadurch wird zudem die Geräuschentwicklung deutlich reduziert und die Pumpe 1 läuft beinahe geräuschlos.

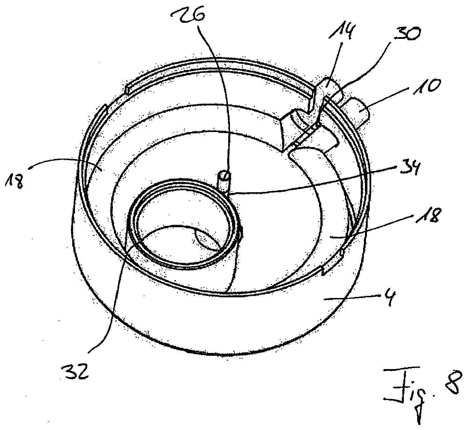

Eine vereinfachte Darstellung aus einem etwas anderen Blickwinkel ist in Figur 8 gezeigt. Das erste Schlauchende 10 ist durch die Öffnung 30 geführt. Im Innenraum des Gehäuses 2 macht der Schlauch in Figur 8 eine Linkskurve und wird an der Innenwand der Umfangswandung 4 entlang geführt. Nachdem er wieder die Öffnung 30 erreicht hat, wird das zweite Schlauchende 14 durch die Öffnung 30 heraus geführt. In der in Figur 8 gezeigten Darstellung ist gut zu erkennen, dass es in dieser Ausführungsform zu keiner Überlagerung oder Überschneidung des Schlauchs 18 mit sich selbst kommt. Es handelt sich vielmehr um eine plane Schlaufe, wodurch gewährleistet ist, dass der Schlauch 18 genau einmal um die Welle 26 an der Innenwand der Umfangswandung 4 entlang geführt wird, ohne dass er insbesondere im Bereich der Öffnung 30 übereinander geführt werden muss. Damit auch in dieser Ausführungsform gewährleistet ist, dass durch den Schlauch 18 keine Luft in das bereits ausgepumpte Volumen zurückströmen kann, muss die Rolle 32 im Bereich der Öffnung 30 den Schlauch 18 sowohl im Bereich des ersten Schlauchendes 10 als auch im Bereich des zweiten Schlauchendes 14 abdrücken. Daher ist es von Vorteil, wenn beide Schlauchenden 10, 14 möglichst dicht beieinander aus dem Gehäuse 2 hinaus beziehungsweise in das Gehäuse 2 hinein geführt werden.

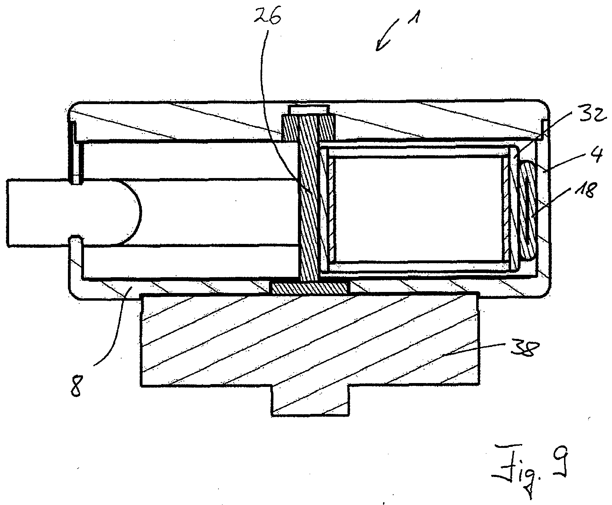

Figur 9 zeigt eine schematische Schnittdarstellung durch eine peristaltische Pumpe 1, wie sie in den Figuren 5, 6, 7 und 8 dargestellt ist. Auch hier bilden die Welle 26, die Rolle 32 und der Schlauch 18 ein Reibradplanetengetriebe, so dass die Rolle 32 nicht über eine Rollenachse 22 verfügt. Das nötige Drehmoment wird allein durch den Reibkontakt zwischen der Rolle 32 und der Welle 26 übertragen. Zwischen der Rolle 32 und der Umfangswandung 4 des Gehäuses 2 der peristaltischen Pumpe 1 wird der Schlauch 18 abgequetscht. Unterhalb des Bodens 8 des Gehäuses 2 ist ein Motor 38 gezeigt, der beispielsweise als Scheibenläufermotor ausgebildet ist und die Welle 26 antreibt.

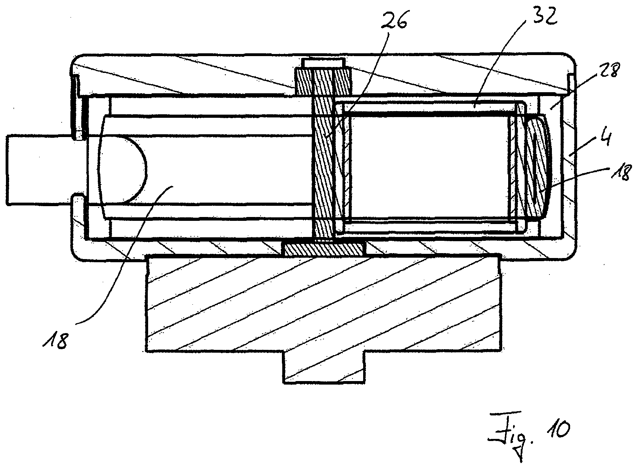

Die gleiche Darstellung ist in Figur 10 gezeigt, mit dem Unterschied, dass an der Innenwandung der Umfangswandung 4 eine Führung 28 angeordnet ist, die ein Verschieben und Verrutschen des Schlauchs 18 in bezüglich der Welle 26 axialer Richtung, in Figur 10 also nach oben oder unten, verhindert. Die Führung 28, die beispielsweise in Form eines Silikonkissens ausgebildet sein kann, dient jedoch nicht nur zum Führen des Schlauches, sondern auch als Toleranzausgleich. Durch die Elastizität der Führung 28 können beispielsweise Fertigungstoleranzen oder unterschiedliche Ausdehnungen der beteiligten Bauteile bei Temperaturänderungen ausgeglichen werden. Auch wenn der Schlauch 18 beispielsweise durch Abnutzung an Durchmesser verliert, kann dies durch die Führung 28 ausgeglichen werden. Gleiches gilt für eine nachlassende Elastizität des Schlauches 18 im Laufe des dauerhaften Betriebes.

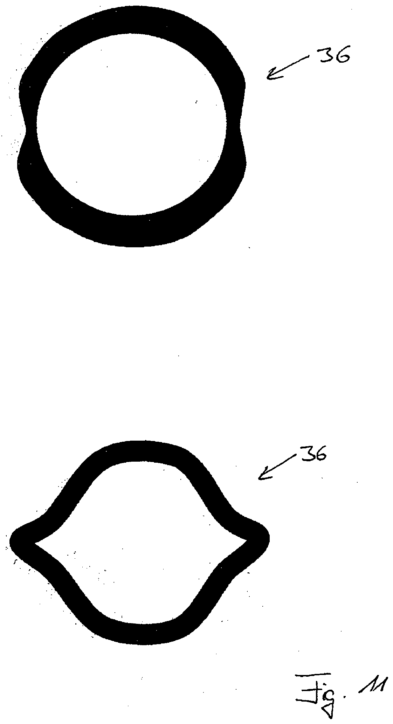

Figur 11 zeigt die schematische Darstellung zweier Schlauchquerschnitte 36. Wird der Schlauch 18 zwischen der Rolle 32 und der Innenwand der Umfangswandung 4 des Gehäuses 2 der peristaltischen Pumpe 1 zusammengequetscht, kommt es insbesondere im Randbereich des Querschnitts zu einer hohen mechanischen Belastung aufgrund der starken Verformung in diesem Bereich. Daher ist in diesem Bereich auch mit dem höchsten Verschleiß der Schläuche 18 bei einer derartigen Pumpenform zu rechnen. Um dies zu verhindern und die Funktionsfähigkeit der peristaltischen Pumpe 1 über einen längeren Zeitraum zu gewährleisten, können bestimmte Schlauchquerschnitte 36 gewählt werden, von denen exemplarisch zwei besonders bevorzugte Ausführungsformen in Figur 11 gezeigt sind. Im oberen Teil von Figur 11 zeigt der Schlauch 18 einen kreisförmigen Schlauchquerschnitt, wobei am rechten und linken Rand die Wandstärke verringert ist. Ein Schlauch mit einem derartigen Querschnitt wird in der peristaltischen Pumpe 1 derart angeordnet, dass der Druck beim Zusammenquetschen von oben nach unten in der in Figur 11 gezeigten Ausrichtung wirkt. Dadurch sind die Verjüngungen in der Wandstärke genau an den Stellen vorgesehen, bei denen die höchste mechanische Belastung auftritt. Da hier weniger Material vorhanden ist, das gequetscht und verformt werden muss, kommt es hier zu weniger starken mechanischen Belastungen, so dass der Verschleiß deutlich verringert wird.

Im unteren Teil der Figur 11 ist ein weiterer Querschnitt 36 gezeigt, der ebenfalls zu einer geringeren mechanischen Belastung insbesondere an den Rändern des zusammengequetschten Schlauchs 18 führt. Auch bei dieser Ausführungsform ist der Schlauch 18 so im Gehäuse 2 der peristaltischen Pumpe 1 anzuordnen, dass der Druck in der in Figur 11 gezeigten Ausrichtung von oben nach unten wirkt und den Schlauch folglich in dieser Richtung zusammenquetscht. Es ist leicht vorstellbar, dass bei dem in Figur 11 im unteren Teil gezeigten Querschnitt der Schlauch in dieser Richtung besonders einfach zusammenzudrücken und abzuquetschen ist, so dass es durch die besondere Querschnittsform auch hier in den rechten und linken Randbereichen zu einer deutlich weniger starken mechanischen Belastung kommt. Dadurch wird auch bei diesem Querschnitt die Lebensdauer des Schlauches und damit die Funktionsfähigkeit der peristaltischen Pumpe 1 deutlich verlängert.

Durch die gewählte Form des Querschnitts 36 wird eine Gestaltfestigkeit erreicht, da der Schlauch 18 nach dem Abdrücken durch das Pumpelement 20 wieder seine ursprüngliche Form annimmt. Zudem wird der Verschleiß an dem beim Abdrücken besonders beanspruchten Stellen vermindert.

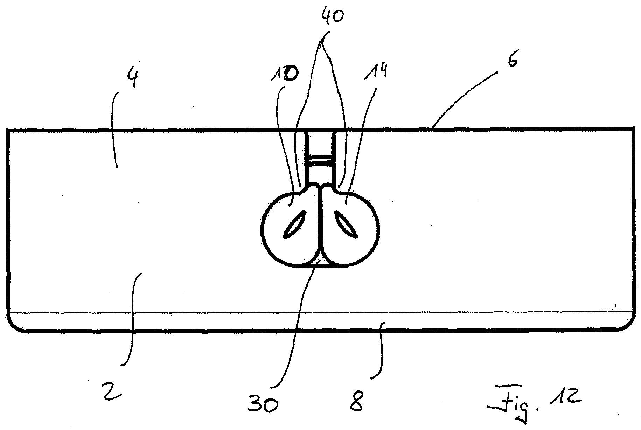

Figur 12 zeigt eine schematische Seitenansicht der peristaltischen Pumpe gemäß einem Ausführungsbeispiel der vorliegenden Erfindung. Das Gehäuse 2 weist den Deckel 6, den Boden 8 und die Umfangswandung 4 auf. In der Umfangswandung 4 befindet sich die Öffnung 30, durch die das erste Schlauchende 10 und das zweite Schlauchende 14 geführt sind.

Man erkennt in Figur 12, dass die Öffnung 30 oval ausgebildet ist und im oberen Bereich einen Steg 40 aufweist. Man erkennt, dass das erste Schlauchende 10 und das zweite Schlauchende 14 in diesem Bereich eine leichte Wulst ausbilden. Durch den Steg 40 wird das erste Schlauchende 10 und das zweite Schlauchende 14 in die in Figur 12 gezeigte Position gedrückt, so dass beide Schlauchenden 10, 14 leicht geöffnet sind, so dass durch den Schlauch ein Medium, beispielsweise Luft, gepumpt werden kann. Bei der Wahl der Form der Öffnung 30 ist daher darauf zu achten, dass zum einen das erste Schlauchende 10 und das zweite Schlauchende 14 nicht vollständig abgequetscht werden, so dass ein Medium durch den Schlauch 18 gepumpt werden kann. Gleichzeitig muss jedoch die Öffnung 30 so klein gewählt werden, dass das erste Schlauchende 10 und das zweite Schlauchende 14 so dicht beieinander angeordnet werden, dass eine innerhalb des Gehäuses 2 der peristaltischen Pumpe 1 umlaufendes Pumpelement 20, beispielsweise die Rolle 32, beide Schlauchenden 10, 14 gleichzeitig abdrücken kann, um einen Rückfluss des Mediums zu vermeiden.

Beide Kriterien sind in der in Figur 12 gezeigten Form der Öffnung 30 erfüllt. Würde die Öffnung 30 kleiner ausgebildet, würde das erste Schlauchende 10 und das zweite Schlauchende 14 allein durch die Öffnung 30 vollständig abgeklemmt, so dass ein Medium nicht mehr hindurch gefördert werden könnte. Wäre die Öffnung 30 größer ausgebildet, könnte ein innerhalb der Pumpe 1 umlaufendes Pumpelement 20 nicht mehr beide Schlauchenden 10, 14 gleichzeitig abdrücken.

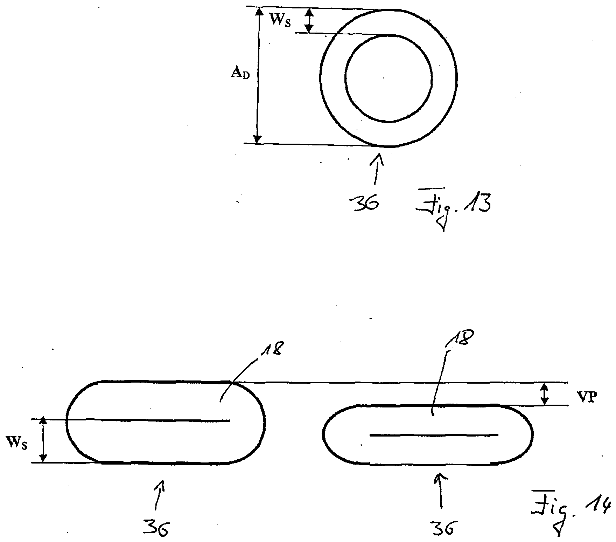

Figur 13 zeigt eine Darstellung des schematischen Schlauchquerschnitts 36. Mit $A_D$ wird dabei der Außendurchmesser des Schlauches bezeichnet, während $W_S$ die Wandstärke des Schlauches darstellt. Die Wandstärke $W_S$ des Schlauches 18 liegt vorteilhafterweise zwischen 0,5 und 1,5 mm.

In Figur 14 ist der Schlauchquerschnitt 36 gezeigt. Dabei ist im linken Teil von Figur 14 der Schlauchquerschnitt 36 so zusammengedrückt, dass eine obere und eine untere Wand des Schlauches 18 aufeinander aufliegen, so dass kein Fluid mehr durch den Schlauch 18 hindurch geführt werden kann. Die Gesamthöhe des so zusammen gelegten Schlauches 18 entspricht zweimal der Wandstärke $W_S$. Eine zusätzliche Quetschung des Schlauchmaterials findet in diesem Beispiel nicht statt. Im rechten Teil der Figur 14 hingegen ist der Schlauch 18 in dem Zustand dargestellt, in den er durch ein umlaufendes Pumpelement 20 gebracht wird. Man erkennt, dass der Schlauch 18 weiter zusammengedrückt wird als für das Schließen des Schlauchquerschnittes 36 eigentlich notwendig wäre. Die Differenz zwischen dem im linken Teil gezeigten lediglich leicht geschlossenen Schlauchs 18 und dem zusammen gepressten in Figur 14 rechts dargestellten Schlauch ist mit VP gekennzeichnet und bezeichnet die Verpressung des Schlauches. Diese beträgt in einer bevorzugten Ausführungsform zwischen 20 und 25 %. Im in Figur 14 gezeigten Ausführungsbeispiel entspricht die Verpressung VP also 20 % bis 25 % der zweifachen Wandstärke $W_S$.

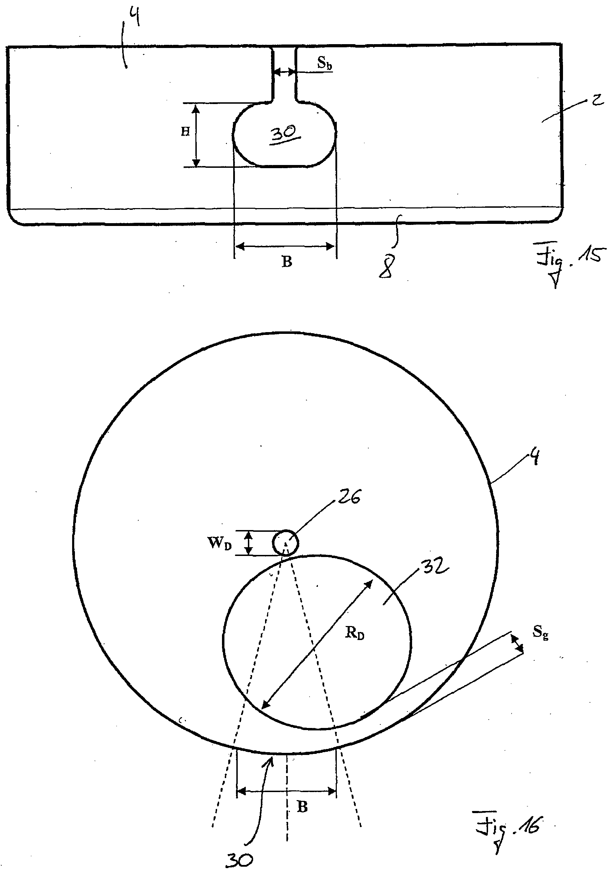

Figur 15 zeigt eine Seitenansicht des Gehäuses 2 ganz ähnlich zu der in Figur 12 gezeigten Ansicht. Man erkennt an dem Gehäuse 2 die Umfangswandung 4 sowie den Boden 8. In der Umfangswandung 4 ist die Öffnung 30 dargestellt, durch die der Schlauch 18, der in Figur 15 nicht gezeigt ist, in das Gehäuse 2 hinein und aus ihm heraus geführt wird.

Die hier beschriebenen Abmessungen für den verwendeten Schlauch 28 sowie die darauf abgestimmten Maße des Gehäuses 2 und der restlichen Bauteile der peristaltischen Pumpe 1 sind angepasst auf eine Ausführungsform, bei der der Schlauch 18 TPE-Schlauch ist. Sie bilden lediglich besonders bevorzugte Ausgestaltungen und Abmessungen der einzelnen Bauteile.

Figur 15 zeigt Abmessungen der Öffnung 30 sowie des Stegs 40. Die Höhe der Öffnung 30 wird durch H dargestellt. Die Höhe H ist bevorzugt etwas kleiner als der Außendurchmesser $A_D$ des Schlauches. Bevorzugt gilt: H = $A_D$/1,25.

Mit B wird die Breite der Öffnung 30 benannt. Da der Schlauch 18 zweimal durch die Öffnung 30 geführt werden muss, ist die Breite B bevorzugt größer als der Außendurchmesser $A_D$, jedoch kleiner als der zweifache Außendurchmesser $A_D$. Bevorzugt gilt für die Breite B: B = $A_D$/0,83.

Die Breite des Steges 40 wird mit $S_b$ gekennzeichnet. Für Sie gilt bevorzugt:

$$S_b = VP*2*W_s.$$

Figur 16 zeigt eine schematische Draufsicht auf das Pumpengehäuse 2, von dem der Deckel 6 entfernt wurde. Man erkennt die Umfangswandung 4, die die in der Mitte angeordnete Welle 26 umgibt. Zwischen dieser und dem an der Umfangswandung 4 verlaufenden Schlauch 18, der in Figur 16 nicht dargestellt ist, erstreckt sich die Rolle 32. In Figur 16 ist zudem die Breite B der Öffnung 30 dargestellt. Die Rolle 32 weist einen Rollendurchmesser $R_D$, während die Welle 26 einen Wellendurchmesser $W_D$ aufweist. Damit ergibt sich das Untersetzungsverhältnis bei angetriebener Welle 26 zu $W_D/R_D$.

Dabei ist der Radius der Welle bei gegebenem Rollendurchmesser $R_D$, gegebenem Außendurchmesser $A_D$ des Schlauches sowie gegebenem Innendurchmesser der Umfangswandung 4 gegeben durch folgende Relation: Wellenradius $W_R$ = Gehäuseinnendurchmesser minus Stärke des gestauchten Schlauches minus Rollendurchmesser $R_D$. Der Durchmesser des gestauchten Schlauches ist gleich zweimal der Wandstärke $W_s$ multipliziert mit 1 minus der Verpressung VP.

Eine zentrale Bedingung für die Größe der zu wählenden Öffnung 30 stellt das Verhältnis des Rollendurchmessers $R_D$ zur Breite B der Öffnung 30 dar. In einer bevorzugten Ausführungsform beträgt das Verhältnis von Rollendurchmesser $R_D$ zur Breite B der Öffnung 30 2,34, sodass der Rollendurchmesser $R_D$ 2,34 mal der Breite B der Öffnung 30 entspricht.

Natürlich sind funktionierende peristaltische Pumpen 1 für Prothesen gemäß einem Ausführungsbeispiel der vorliegenden Erfindung auch mit anderen Abmessungen, Maßen und Materialien vorstellbar.

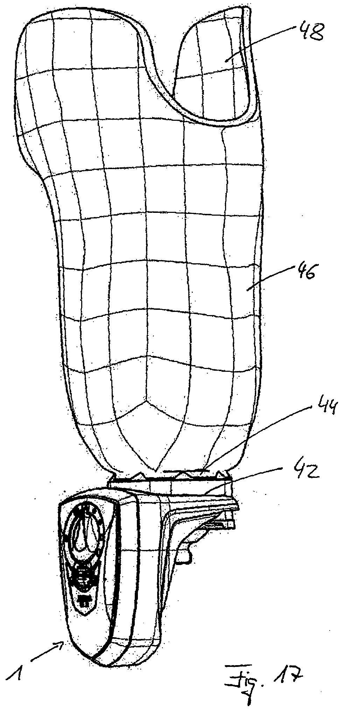

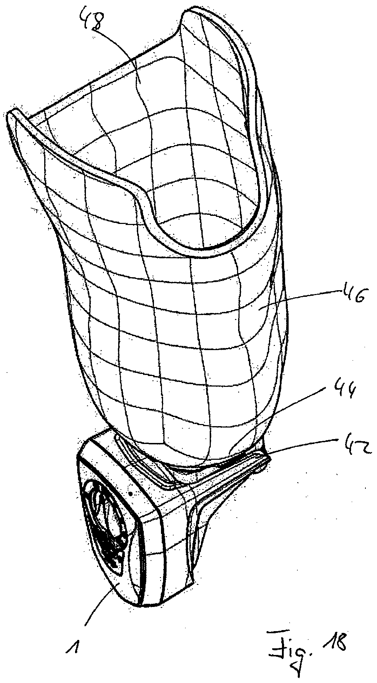

Figur 17 zeigt eine schematische 3D-Ansicht einer peristaltischen Pumpe 1, die an einer Pumpenhalterung 42 angeordnet ist, die an einem distalen Ende 44 eines Prothesenschaftes 46 angeordnet ist. Im oberen Bereich erkennt man, dass der Prothesenschaft 46 über eine Innenfläche 48 verfügt, mit der der Schaft 46 an einem nicht gezeigten Amputationsstumpf anliegt. Figur 18 zeigt die Darstellung aus Figur 17 in einem leicht veränderten Blickwinkel.

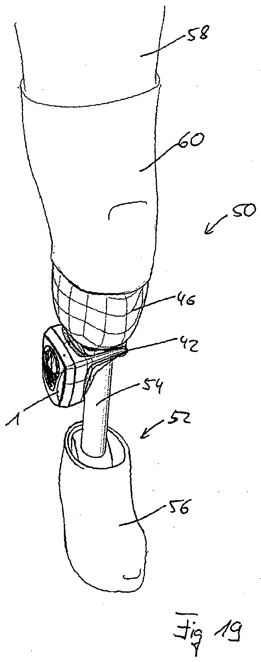

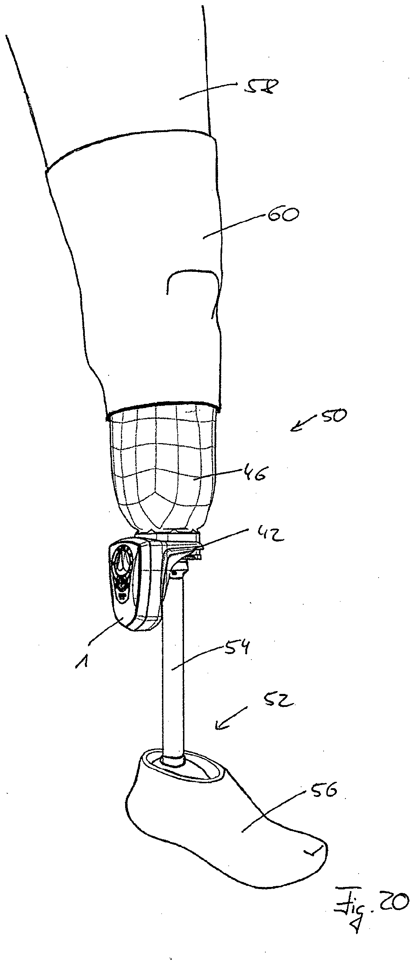

Die Figuren 19 und 20 zeigen eine Prothese 50 gemäß einem Ausführungsbeispiel der vorliegenden Erfindung aus zwei leicht unterschiedlichen Blinkwinkeln. Man erkennt die peristaltische Pumpe 1, die über die Pumpenhalterung 42 am Prothesenschaft 46 angeordnet ist. Am distalen Ende befindet sich eine Protheseneinrichtung 52, die über ein Rohrelement 54 und ein an dessen distalen Ende angeordnetes Fußelement 56 verfügt. Am jeweils oberen Rand der Figuren 19 und 20 ist ein Amputationsstumpf 58 zu erkennen, der in dem Prothesenschaft 46 angeordnet ist. Über die Verbindungsstelle zwischen Prothesenschaft 46 und Amputationsstumpf 58 ist eine so genannte Knieknappe 60 bzw. ein Sleeve gezogen. Diese Kniekappe 60 sorgt für einen luftdichten Abschluss des zwischen dem Prothesenschaft 46 und dem Amputationsstumpf 58 eingeschlossenen Volumens. Natürlich ist es auch denkbar, beispielsweise an der Innenfläche 48 des Prothesenschaftes 46 ein Dichtelement anzubringen, um das zwischen dem Prothesenschaft 46 und dem Amputationsstumpf 58 befindliche Volumen luftdicht abzuschließen. Alternativ oder zusätzlich dazu kann auch ein Liner vorgesehen sein, der über den Amputationsstumpf 58 gezogen wird, bevor dieser in den Prothesenschaft 46 eingefügt wird.

### Bezugszeichenliste

| | | | |
|---|---|---|---|
| 1 | peristaltische Pumpe | 56 | Fußelement |
| 2 | Gehäuse | 58 | Amputationsstumpf |
| 4 | Umfangswandung | 60 | Kniekappe |
| 6 | Deckel | $A_D$ | Außendurchmesser |
| 8 | Boden | | des Schlauches |
| 10 | Erstes Schlauchende | $W_s$ | Wandstärke |
| 12 | Erste Öffnung | VP | Verpressung |
| 14 | Zweites Schlauchende | H | Höhe |
| 16 | Zweite Öffnung | B | Breite |
| 18 | Schlauch | $S_b$ | Stegbreite |
| 20 | Pumpelement | $R_D$ | Rollendurchmesser |
| 22 | Rollenachse | $W_D$ | Wellendurchmesser |
| 24 | Antriebsscheibe | $W_R$ | Wellenradius |
| 26 | Welle | | |
| 28 | Führung | | |
| 30 | Öffnung | | |
| 32 | Rolle | | |
| 34 | Aufgerauter Bereich | | |
| 36 | Schlauchquerschnitt | | |
| 38 | Motor | | |
| 40 | Steg | | |
| 42 | Pumpenhalterung | | |
| 44 | Distales Ende | | |
| 46 | Prothesenschaft | | |
| 48 | Innenfläche | | |
| 50 | Prothese | | |
| 52 | Protheseneinrichtung | | |
| 54 | Rohrelement | | |

**Patentansprüche**

1.  Prothese (50) mit
    einem Prothesenschaft (46), der

    eine Innenfläche (48) aufweist und
    ausgebildet ist, an einem Amputationsstumpf (58) angeordnet zu werden, so dass

    die Innenfläche (48) dem Amputationsstumpf (58) zugewandt ist und

zwischen der Innenfläche (48) und dem Amputationsstumpf (58) ein Volumen eingeschlossen ist, und

einer Pumpe zum Erzeugen eines Unterdrucks in dem Volumen, wenn der Prothesenschaft (46) an dem Amputationsstumpf (58) angeordnet ist, **dadurch gekennzeichnet, dass** die Pumpe eine peristaltische Pumpe (1) ist.

2. Prothese (50) nach Anspruch 1, **dadurch gekennzeichnet, dass** die peristaltische Pumpe (1) ein Gehäuse (2) mit einer Umfangswandung (4), einem Boden (8) und einem Deckel (6) umfasst, in dem genau ein Pumpelement (20) angeordnet ist.

3. Prothese (50) nach Anspruch 2, **dadurch gekennzeichnet, dass** in der Umfangswandung (4) eine Öffnung (30) vorgesehen ist, durch die ein Schlauch (18) in das Gehäuse (2) hinein und aus dem Gehäuse (2) heraus geführt ist.

4. Prothese (50) nach Anspruch 3, **dadurch gekennzeichnet, dass** in dem Gehäuse (2) der Schlauch (18) so geführt ist, dass es an keiner Stelle zu einer Überlagerung oder Überschneidung des Schlauches (18) mit sich selbst kommt.

5. Prothese (50) nach einem der Ansprüche 2 bis 4, **dadurch gekennzeichnet, dass** das Pumpelement (20) eine Rolle (32) ist, die so in dem Gehäuse (2) angeordnet ist, dass sie um eine Welle (26) umlaufen kann und den Schlauch (18) abklemmt.

6. Prothese (50) nach Anspruch 5, **dadurch gekennzeichnet, dass** der Schlauch (18) an einer Innenseite der Umfangswandung (4) entlang geführt ist und mit der Rolle (32) und der Welle (26) ein Reibradplanetengetriebe bildet.

7. Prothese (50) nach Anspruch 5 oder 6, **dadurch gekennzeichnet, dass** die Rolle (32) eine Hohlrolle ist.

8. Prothese (50) nach einem der Ansprüche 2 bis 7, **dadurch gekennzeichnet, dass** an einer Innenseite des Deckels (6) und/oder einer Innenseite des Bodens (8) des Gehäuses (2) eine reibungsmindernde Schicht, insbesondere eine PTFE-Gewebefolie angeordnet ist.

9. Prothese (50) nach einem der Ansprüche 3 bis 8, **dadurch gekennzeichnet, dass** der Schlauch (18) aus Silikon oder einem thermoplastischen Elastomer oder aus Polyurethan besteht.

10. Prothese (50) nach einem der vorstehenden Ansprüche, **dadurch gekennzeichnet, dass** sie einen Motor zum Antreiben der Pumpe (1) umfasst, der in Form eines Scheibenläufermotors ausgebildet ist.

**Claims**

1. Prosthesis (50) with a prosthesis socket (46) which has an inner face (48) and is designed to be arranged on an amputation stump (58), such that the inner face (48) is directed toward the amputation stump (58) and a volume is enclosed between the inner face (48) and the amputation stump (58), and with a pump for generating an underpressure in the volume when the prosthesis socket (46) is arranged on the amputation stump (58), **characterized in that** the pump is a peristaltic pump (1).

2. Prosthesis (50) according to Claim 1, **characterized in that** the peristaltic pump (1) comprises a housing (2) which has a circumferential wall (4), a floor (8) and a lid (6) and in which exactly one pump element (20) is arranged.

3. Prosthesis (50) according to Claim 2, **characterized in that** an opening (30) through which a hose (18) is guided into the housing (2) and out of the housing (2) is provided in the circumferential wall (4).

4. Prosthesis (50) according to Claim 3, **characterized in that** the hose (18) is guided in the housing (2) in such a way that at no point does the hose (18) overlap itself.

5. Prosthesis (50) according to one of Claims 2 through 4, **characterized in that** the pump element (20) is a roller (32), which is arranged in the housing (2) such that it can rotate about a shaft (26) and pinches the hose (18) shut.

6. Prosthesis (50) according to Claim 5, **characterized in that** the hose (18) is guided along an inner side of the circumferential wall (4) and forms a frictional planetary gear with the roller (32) and the shaft (26).

7. Prosthesis (50) according to Claim 5 or 6, **characterized in that** the roller (32) is a hollow roller.

8. Prosthesis (50) according to one of Claims 2 through 7, **characterized in that** a friction-reducing layer, in particular a PTFE fabric sheet, is arranged on an inner side of the lid (6) and/or on an inner side of the floor (8) of the housing (2).

9. Prosthesis (50) according to one of Claims 3 through 8, **characterized in that** the hose (18) is made of silicone or of a thermoplastic elastomer or of polyurethane.

10. Prosthesis (50) according to one of the preceding claims, **characterized in that** it comprises a motor for driving the pump (1), which motor is designed in the form of a disk rotor motor.

**Revendications**

1. Prothèse (50) comprenant
une emboîture de prothèse (46) qui
comporte une surface intérieure (48), et
est réalisée pour être agencée sur un moignon d'amputation (58) de telle sorte que

- la surface intérieure (48) est tournée vers le moignon d'amputation (58) et
- un volume est enfermé entre la surface intérieure (48) et le moignon d'amputation (58), et

une pompe pour engendrer une dépression dans le volume quand l'emboîture de prothèse (46) est agencée sur le moignon d'amputation (58),
**caractérisée en ce que** la pompe est une pompe péristaltique (1).

2. Prothèse (50) selon la revendication 1, **caractérisée en ce que** la pompe péristaltique (1) inclut un boîtier (2) avec une paroi périphérique (4), un fond (8) et un couvercle (6), dans lequel est agencé exactement un élément de pompe (20).

3. Prothèse (50) selon la revendication 2, **caractérisée en ce qu'**une ouverture (30) est prévue dans la paroi périphérique (4), ouverture à travers laquelle est passé un tuyau (18) pour entrer dans le boîtier (2) et sortir hors du boîtier (2).

4. Prothèse (50) selon la revendication 3, **caractérisée en ce que** le tuyau (18) est guidé dans le boîtier (2) de telle façon qu'il ne se produit à aucun endroit une superposition ou un recoupement du tuyau (18).

5. Prothèse (50) selon l'une des revendications 2 à 4, **caractérisée en ce que** l'élément de pompe (20) est un galet (32) qui est agencé dans le boîtier (2) de telle façon qu'il est capable de circuler autour d'un arbre (26) et pince le tuyau (18).

6. Prothèse (50) selon la revendication 5, **caractérisée en ce que** le tuyau (18) est guidé le long d'une face intérieure de la paroi périphérique (4) et forme avec le galet (32) et avec l'arbre (26) un mécanisme planétaire à roulettes de friction.

7. Prothèse (50) selon la revendication 5 ou 6, **caractérisée en ce que** le galet (32) est un galet creux (32).

8. Prothèse (50) selon l'une des revendications 2 à 7, **caractérisée en ce qu'**une couche réduisant la friction, en particulier un film textile en PTFE, est agencée sur une face intérieure du couvercle (6) et/ou sur une face intérieure du fond (8) du boîtier (2).

9. Prothèse (50) selon l'une des revendications 3 à 8, **caractérisée en ce que** le tuyau (18) est en silicone ou en élastomère thermoplastique ou encore en polyuréthane.

10. Prothèse (50) selon l'une des revendications précédentes, **caractérisée en ce qu'**il inclut un moteur pour entraîner la pompe (1), qui est réalisé sous la forme d'un moteur à rotor en forme de disque.

Fig. 1

EP 2 599 464 B1

Fig. 2

Fig. 3

Fig. 4

Fig. 5

Fig. 6

Fig. 7

EP 2 599 464 B1

Fig. 8

Fig. 9

Fig. 11

26    32

28

4

18

18

*Fig. 10*

Fig. 12

Fig. 13

Fig. 14

Fig. 15

Fig. 16

Fig. 17

Fig 19

Fig. 18

58

60

50

46

42

1

54

52

56

Fig. 20

**IN DER BESCHREIBUNG AUFGEFÜHRTE DOKUMENTE**

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Patentdokumente**

- US 20070191965 A1
- WO 2006135851 A2
- US 5702489 A
- US 6926742 B
- US 20030181990 A1